Europäisches Patentamt

European Patent Office

Office européen des brevets

(19)

(11) Veröffentlichungsnummer: **0 419 446 B1**

(12) **EUROPÄISCHE PATENTSCHRIFT**

(45) Veröffentlichungstag der Patentschrift: **19.04.95**

(51) Int. Cl.6: **C07K  1/04**, C07K 17/00, A61K 39/29, G01N 33/569

(21) Anmeldenummer: **90890262.0**

(22) Anmeldetag: **13.09.90**

(54) **Komplex geeignet zur Durchführung eines Verfahrens zur Reinigung von pre-S-Hepatitis-B-Virus-Oberflächenantigen.**

(30) Priorität: **20.09.89 AT 2198/89**

(43) Veröffentlichungstag der Anmeldung:
**27.03.91 Patentblatt  91/13**

(45) Bekanntmachung des Hinweises auf die
Patenterteilung:
**19.04.95 Patentblatt  95/16**

(84) Benannte Vertragsstaaten:
**AT BE CH DE DK ES FR GB IT LI LU NL SE**

(56) Entgegenhaltungen:
DE-A- 2 530 247
US-A- 4 855 055

BIOLOGICAL ABSTRACTS, Band 84, Nr. 7,
1987, Seite AB-596, Zusammenfassung
Nr.67918, Philadelphia, PA, US; Y. ISE et al.:
"Interaction of hepatitis B surface

antigen with serum albumin of various species on polystyrene latex particles",& MED.
MICROBIOL. IMMUNOL. 176(4): 199-208. 1987

ARCHIVES OF VIROLOGY, Band 98, Nr. 3/4,
1988, Seiten 163-170, Springer-Verlag,Wien,

AU; L. XUANYONG et al.: "The interaction
between native serum albumin andhepatits
B virus"

(73) Patentinhaber: **IMMUNO Aktiengesellschaft
Industriestrasse 67
A-1221 Wien (AT)**

(72) Erfinder: **Eibl, Johann, Dr.
Gustav Tschermakgasse 2
A-1180 Wien (AT)**
Erfinder: **Dorner, Friedrich, Prof.Dr.
Peterlinigasse 17
A-1230 Wien (AT)**
Erfinder: **Mitterer, Arthur, Dr.
Am Markt 30
A-2304 Orth/Donau (AT)**

(74) Vertreter: **Wolfram, Gustav, Dipl.-Ing.
Patentanwälte
Sonn, Pawloy, Weinzinger & Wolfram,
Riemergasse 14
A-1010 Wien (AT)**

**Beschreibung**

Die Erfindung betrifft einen Komplex, bestehend aus einem unlöslichen polymeren Träger, an dem Humanalbumin in kovalent gebunden ist, und einem pre-S-Hepatitis-B-Virus-Oberflächenantigen (pre-S-HBsAg), sowie ein Verfahren zur Reinigung von pre-S-HBsAg.

Beispiele für wasserunlösliche Proteinpräparate, die aus einem polymeren wasserunlöslichen Träger bestehen, an den über ein Zwischenglied Enzyme oder Antikörper kovalent gebunden sind, werden in der DE-A-2 530 247 beschrieben.

Aus der EP-A2 - 0 243 103 ist ein Verfahren zur Reinigung von pre-S-HBsAg bekannt, welches darin besteht, daß Hefezellen, die rekombiniertes pre-S-HBsAg exprimieren, aufgebrochen und aus dem Zellinhalten pre-S-HBsAg affinitätschromatographisch abgetrennt wird. Als Adsorbens für das pre-S-HBsAg fungiert polymerisiertes humanes Serumalbumin (Polyalbumin), das kovalent an eine Matrix gebunden ist. Dieses Polyalbumin ist ein durch Quervernetzer, z.B. Glutaraldehyd, in hochmolekularer Form künstlich hergestelltes Produkt. Das pre-S-HBsAg adsorbiert über seinen pre-S(2)-Teil am Polyalbumin und wird, nachdem störende Substanzen ausgewaschen wurden, von der Matrix eluiert und einem weiteren Reinigungsschritt unterworfen.

Auch aus der US-A-4 855 055 ist ein Verfahren zur Isolierung und affinitätschromatographischen Reinigung von pre-S-Hepatitis-B-Oberflächenantigen unter Verwendung von polymeren Humanalbumin bekannt.

Es wurde nachgewiesen, daß im pre-S-Teil des HBsAg ein effizienter Rezeptor für Polyalbumin vorhanden ist, der aus einem Polypeptid mit 55 Aminosäuren besteht und von einem Abschnitt der Hepatitis-Virus-DNA kodiert wird, der dem S-Bereich unmittelbar vorgelagert ist ("pre-S2") (Valenzuela et al., Bio/Technology 3: 317-320, 1985). Es wurde weiters nachgewiesen, daß sowohl dieser unmittelbar vorgelagerte Bereich (pre-S2) als auch der gesamte pre-S-Bereich in Verbindung mit dem S-Bereich (pre-S1) Oberflächenproteine kodieren, die Rezeptoren des Hepatitis-B-Virus (HBV) für die Bindung an die Zellmembran von Leberzellen darstellen.

HBsAg, das einen pre-S-Teil enthält, wurde erstmals 1979 aus infiziertem menschlichem Plasma gewonnen (Neurath und Strick, Arch. Virol. 60: 79-81, 1979) und wurde später von Valenzuela et al. (Bio/Technology 3: 317, 1985, und Nature 311: 67, 1984) und Paoletti et al. (PNAS 81: 193, 1984) auch auf gentechnologischem Weg hergestellt.

In der Folge wurden verschiedene rekombinant exprimierte pre-S-haltige Oberflächen-Antigene als Impfstoff zur Induzierung der Antikörperbildung gegen das HBV vorgeschlagen. Diese Antikörper richten sich gegen den Rezeptor des Virus und verhindern so seine Bindung an die Leberzelle und damit die Infektion.

Die affinitätschromatographische Reinigung der Antigene über Polyalbumin hat den Nachteil, daß durch die Verwendung der Quervernetzer bei der Herstellung der Polymerisationsprodukte Verunreinigungen, sogar toxische Substanzen, wie Glutaraldehyd, in das Eluat eingebracht werden.

Die vorliegende Erfindung setzt sich zum Ziel, diese Schwierigkeit zu beseitigen und einen Komplex zur Verfügung zu stellen, der neben der Möglichkeit einer schnellen und effizienten affinitätschromatographischen Reinigung von pre-S-HBsAg Einsatzmöglichkeiten für therapeutische und diagnostische Zwecke eröffnet.

Der erfindungsgemäße Komplex besteht aus einem unlöslichen polymeren Träger, insbesondere auf Agarose- oder Dextranbasis, an welchem Träger monomeres Humanalbumin kovalent gebunden ist, und einem pre-S-Hepatitis-B-Virus-Oberflächenantigen, welches über seinen pre-S(2)- und/oder pre-S(1)-Teil an dem monomeren Humanalbumin in eluierbarer Form gebunden ist.

Erfindungsgemäß wurde festgestellt, daß das pre-S-HBsAg nur dann in einer für eine affinitätschromatographische Reinigung ausreichenden Stärke an das monomere Humanalbumin komplexiert, wenn dieses kovalent an den Träger gebunden ist. Wird das Album in lediglich adsorptiv gebunden, kann das pre-S-HBsAg nicht bzw. nur äußerst schwach an das Album in komplexiert werden, so daß eine affinitätschromatographischen Reinigung nicht ermöglicht wird. Ein weiterer Vorteil des erfindungsgemäßen Komplexes besteht darin, daß das pre-S-HBsAg leicht eluierbar ist, wodurch das Protein schonender und in höherer Ausbeute gewonnen werden kann.

Für die Bildung des erfindungsgemäßen Komplexes ist ein unlöslicher polymerer Träger, an dem monomeres Humanalbumin kovalent gebunden ist, erforderlich. Dieser unlösliche polymere Träger fällt daher auch in den Rahmen der Erfindung. Zur Herstellung des erfindungsgemäßen unlöslichen polymeren Trägers kann jedes Polymer verwendet werden, das nach entsprechender Aktivierung zur kovalenten Bindung von Proteinen befähigt ist. Als Trägermaterial kommen

- organische Polymere, wie Polyamide und Vinylpolymere (Polyacrylamid, Polystyrol und Polyvinylalkohole und deren Derivate), weiters
- natürliche Polymere, wie Cellulose, Dextrane, Agarose, Chitin und Polyaminosäuren, und
- anorganische Polymere, wie Kieselgel, Glas und Metallhydroxide,

in Frage. Diese Trägermaterialien können in Form von Partikeln, z.B. als Molekularsieb, in Form von Membranen oder von Platten, z. B. als Mikrotiterplatten, verwendet werden.

Vorzugsweise ist der unlösliche polymere Träger auf Agarose-oder Dextranbasis aufgebaut.

Der erfindungsgemäße Komplex kann über längere Zeit gelagert werden, u.zw. sowohl in wässeriger Phase, in der der Träger in gequollenem Zustand vorliegt (z.B. als Affinitätsharz), als auch in lyophilisiertem Zustand (z.B.als Membran oder Mikrotiterplatte). Eine Lyophilisierung wird vorzugsweise aus einem flüchtigen Puffer durchgeführt, der Glycin oder Glukose enthält.

Aus dem erfindungsgemäßen Komplex kann das pre-S-HBsAg in hoher Reinheit isoliert werden. Die Erfindung betrifft auch die Verwendung des erfindungsgemäßen Komplexes zur Herstellung von Diagnostika und Impfstoffen, wobei die Impfstoffe sowohl zur aktiven Immunisierung gegen Hepatitis B als auch zur Gewinnung von spezifischem Immunglobulin von Spendern, die mit solchen Impfstoffen immunisiert wurden, eingesetzt werden können.

Der erfindungsgemäße Träger kann auf einfache Weise mit pre-S-Hepatitis-B-Virus-Oberflächenantigen beladen werden, indem eine wässerige Lösung von Hepatitis-B-Virus-Oberflächenantigen mit dem Träger in Kontakt gebracht wird, wobei selektiv die pre-S-hältigen Anteile des Hepatitis-Oberflächenantigens an den Albuminmolekülen adsorbieren.

Das erfindungsgemäße Verfahren zur Reinigung von pre-S-Hepatitis-B-Virus-Oberflächenantigen unter Verwendung eines erfindungsgemäßen Komplexes ist dadurch gekennzeichnet,
- daß der Komplex zur Entfernung von eventuell vorhandenen Verunreinigungen mit Pufferlösung gewaschen wird, und
- daß das am monomeren Humanalbumin selektiv adsorbierte pre-S-Hepatitis-B-Virus-Oberflächenantigen abgespalten und gewonnen wird, indem der Komplex entweder mit einem Elutionsmittel behandelt wird, welches chaotrope Substanzen, wie Harnstoff, Guanidinhydrochlorid, Thiocyanat, Kaliumchlorid, Magnesiumchlorid oder Kaliumjodid, enthält; oder
indem der Komplex mit Detergentien behandelt wird; oder indem der Komplex mit einem den pH-Wert verändernden Agens behandelt wird,
wonach gegebenenfalls ein weiterer Reinigungsschritt vorgenommen wird.

Eine bevorzugte Ausführungsform des erfindungsgemäßen Verfahrens ist dadurch gekennzeichnet, daß als Detergentien ionische Detergentien, insbesondere Natrium-Deoxycholat und Taurodeoxycholsäure, oder zwitterionische Detergentien, insbesondere 3[(3-Cholamidopropyl)-dimethyl-ammonio]-1-propansulfonat und 3[(3-cholamidopropyl)-dimethyl-ammonio]-2-hydroxy-1-propansulfonat, oder nichtionische Detergentien, insbesondere Octyl-Glucopyranoside, eingesetzt werden.

Es hat sich gezeigt, daß bei Verwendung dieser Detergentien die Quartärstruktur des eluierten pre-S-HBsAg nicht beeinträchtigt wird.

Vorzugsweise besteht der weitere Reinigungsschritt nach der Elution in einer Gelfiltration, Ultrazentrifugation, hydrophoben Chromatographie, Lectin-Affinitätschromatographie oder in einer Ionenaustausch-Chromatographie. Jeder einzelne dieser Reinigungsschritte führt zu einer Reinheit des pre-S-HBsAg von über 90 %.

Im folgenden wird die Erfindung näher beschrieben:

Das pre-S-HBsAg kann aus rekombinantem Vaccinia-Virus, der die genetische Information des pre-S-HBsAg enthält, exprimiert werden. Diese Technik ist in der Literatur beschrieben (Moss et al., Nature 311: 67 (1984)).

Zunächst wird eine hochtitrige Virus-Stammlösung, bestehend aus rekombinantem Vaccinia-Virus, hergestellt, indem Vero-Zellen mit 1 pfu/Zelle infiziert werden. Nach 2- bis 3-tägiger Inkubation bei 37°C werden die infizierten Zellen in das Medium überführt und durch 20-minütiges Zentrifugieren bei 5.000 g pelletiert. Der Überstand wird abgegossen und bei 4°C gelagert. Die Zellen werden dreimal mit PBS gewaschen, wonach eine Trypsinlösung zur Zellsuspension zugegeben wird, bis eine Endkonzentration an Trypsin von 0,025 Massen% erreicht ist. Die Suspension wird dann 30 min bei 37°C leicht gerührt, mit dem Überstand vereinigt, in Ampullen aliquotiert und auf -80°C tiefgefroren. Durch dieses Verfahren erhöht sich der Virustiter ungefähr auf den zehnfachen Wert, der sonst im Zellmedium erhalten wird.

Die Vero-Zellen-Startkultur wird bereitet, indem die Zellen in Roux-Flaschen und in Roller-Flaschen (Nunc) aus Kunststoff angezüchtet werden, um ausreichend viele Zellen zu produzieren, um einen 6-Liter-Fermenter bei mpf en zu können, der dann seinerseits als Startkultur für einen 40-Liter-Ansatz dient. Dazu wird zuerst eine einzelne Ampulle mit Vero-Zellen einer bestimmten Generationszahl aufgetaut und weitergezüchtet, um zwölf dichtbewachsene Roller-Flaschen bereitzustellen.

Die Zellen werden trypsinisiert, im Medium 199 mit 5 Massen% fötalem Kalbserum resuspendiert, mit einer Suspension von Mikrocarriern (Cytodex 3, Pharmacia) vermischt und in den Fermenter gepumpt, wobei eine Endkonzentration von $2 \times 10^8$ Zellen und 5 g Mikrocarrier pro Liter erhalten wird. In diesem Stadium wird noch Medium in einer Menge von einem Drittel des End-Arbeitsvolumens zugegeben.

Während drei Stunden wird den Zellen die Möglichkeit gegeben, an die Mikrocarrier zu binden, wobei die Suspension gleichzeitig langsam gerührt wird. Danach wird weiter Medium (DMEM enthaltend 5 Massen% fötales Kalbserum) zugegeben, um das End-Arbeitsvolumen zu erreichen. Sobald eine Zelldichte von 6 bis $8 \times 10^8$/Liter angezüchtet ist, wird eine kontinuierliche Perfusion mit DMEM (enthaltend 5 Massen% fötales Kalbserum) begonnen. Nach Erreichen einer Zelldichte von ungefähr $5 \times 10^9$/Liter werden die Mikrocarrier trypsiniert und die Zellen samt Mikrocarriern in den 40-Liter-Fermenter gepumpt, der pro Liter weitere 5 g Mikrocarrier enthält. Nach der Adsorption wird der Fermenter auf 40 Liter aufgefüllt und die Kultivierung, wie oben beschrieben, fortgeführt.

Wenn die Zelldichte von $5 \times 10^9$/Liter erreicht ist, wird das Medium abgepumpt, nachdem sich die Mikrocarrier abgesetzt haben. Fünf Liter des den rekombinanten Mikroorganismus enthaltenden Mediums werden in den Fermenter gepumpt, um einen m.o.i.-Wert von ungefähr 2 pfu pro rekombinanter Zelle zu ergeben. Nach der Adsorption des Virus wird der Fermenter mit Medium 199 (enthaltend 5 Massen% fötales Kalbserum) auf 40 Liter aufgefüllt und mit 40 Liter desselben Mediums 40 Stunden lang perfundiert. Nach dieser Zeitspanne haben sich ca. 80 % der Zellen von den Mikrocarriern abgelöst und werden mit dem Medium abgepumpt.

Die auf den Mikrocarriern noch restlichen haftenden Zellen werden durch Waschen mit Medium unter schnellem Rühren abgelöst, abgepumpt und mit dem ersten Teil vereinigt. Die Mikrocarrier werden mit Hilfe eines 70 $\mu$m-Siebes entfernt. Durch Zentrifugieren in einem Beckmann JFC-Z-Durchflußrotor werden die Zellen bei 16.000 g pelletiert. Das Medium wird durch Ultrafiltration im Pellikonsystem (Millipore-Waters) konzentriert. Dem konzentrierten Medium wird Harnstoff zugegeben, bis eine 8 M Lösung erreicht ist. Diese Lösung wird dialysiert.

Der konzentrierte Vero-Zellen-Überstand wird einer Säule aufgetragen, die mit einem unlöslichen, polymeren Trägermaterial gefüllt ist, an dem humanes Serumalbumin kovalent gebunden ist. Die Methode der Kopplung richtet sich bekanntlich nach der chemischen Natur des Trägers. Bei Verwendung von Sepharose wird z.B. mit CNBr aktiviert, worauf das monomere humane Serumalbumin an die Sepharose gekoppelt wird.

Nachdem die Säule mit Puffer 1, bestehend aus 0,2 M Natriumacetat (pH 4,0) und 0,5 M NaCl, mit Puffer 2, bestehend aus 0,1 M Tris (pH 8,0) und 0,5 M NaCl, mit einer Lösung 3, bestehend aus 8 M Harnstoff und schließlich mit Puffer 3, bestehend aus 0,02 M Tris (pH 7,4) gewaschen wurde, wird der Vero-Zellen-Überstand der Säule aufgetragen. Sobald der Überstand von der Säule aufgenommen ist, werden die nicht-adsorbierten Proteine mit Puffer 4, bestehend aus 0,02 M Tris (pH 7,4) und 0,5 M NaCl aus der Säule ausgewaschen. Das adsorbierte pre-S(2)-HBsAg wird mit Natriumthiocyanat in Puffer 4 (1 bis etwa 4 M, bevorzugt 3 M) bei einem pH-Wert von 6 bis etwa 8, bevorzugt bei pH 7 (Puffer 5) oder mit 8 M Harnstoff in Puffer 4, bevorzugt mit 4 M Harnstoff (Puffer 6) eluiert. Diese erfindungsgemäße Abtrennung mit monomerem Humanalbumin führt zu einer 145-fachen Anreicherung von pre-S-HBsAg. Seine Identität kann mit der Immunoblot-Technik (Burnette, Anal. Biochem., 112, 195, 1981) und mit mit Silber gefärbten Polyacrylamidgelen ("silver staining"; Morrissey, Anal. Biochem. 117, 307, 1981) nachgewiesen werden, wobei die Reinheit mindestens 80 % beträgt.

Das pre-S(2)-HBsAg kann weiter u.a. durch Gelfiltration auf Sepharose (Pharmacia) gereinigt werden. Dazu wird die oben erhaltene Fraktion gegen einen Puffer, bestehend aus 0,02 M Tris (pH 7) dialysiert und der Sepharose-Säule aufgetragen, die mit 0,02 M Tris (pH 7) aktiviert wurde. Mit den Techniken des "Silver-staining" und des "Immunoblotting" kann eine Reinheit des pre-S-HBsAg von mehr als 90 % nachgewiesen werden.

In den nachfolgenden Ausführungsbeispielen wird die Durchführung des erfindungsgemäßen Verfahrens noch genauer beschrieben:

Beispiel 1:

Affinitätschromatographische Reinigung des pre-S(2)-HBsAg mittels monomeren Humanalbumins

Der rekombinante Vaccinia-Virus mit der genetischen Information für das pre-S(2)-HBsAg wurde mittels der von Moss et al. beschriebenen Techniken erhalten (Nature 311: 67, 1984). Die hochtitrige Stammlösung des rekombinanten Vaccinia-Virus wurde wie oben beschrieben hergestellt. Die Vero-Zellen für die Infektion mit dem rekombinanten Virus wurden unter denselben Bedingungen gezüchtet, wie oben beschrieben. Das konzentrierte, dialysierte Medium mit dem pre-S(2)-HBsAg wurde ebenso erhalten, wie vorstehend beschrieben.

Monomeres Humanalbumin wurde kovalent an CNBr-aktivierte Sepharose 4B (Pharmacia) gebunden, gemäß den Instruktionen des Herstellers. Danach wurde eine Säule mit einem Volumen von 50 ml gefüllt und mit 500 ml der Pufferlösung 1, 500 ml der Pufferlösung 2, 500 ml der Lösung 3 und schließlich 500 ml der Pufferlösung 4 gewaschen.

Der geklärte Vero-Zellen-Überstand enthielt 8500 mg Protein und 95 mg pre-S(2)-HBsAg und wurde in die vorbereitete Säule mit einer Durchflußrate von 100 ml/Stunde gepumpt. Sobald die gesamte Menge der Säule übertragen war, wurden die nicht adsorbierten Proteine mit der Pufferlösung 4 aus der Säule gewaschen. Das pre-S(2)-HBsAg wurde mit 3 M Natriumthiocyanat, pH 7 (Pufferlösung 5) eluiert. Die Identifizierung und die Reinheitsprüfung erfolgte mit der "silver-staining"-Methode und Immunoblotting, nach einer Polyacrylamid-Gelelektrophorese.

Die von der Säule eluierten Fraktionen wurden zweigeteilt und 15 min bei 100°C in einem Puffer, bestehend aus 2 Massen% Natriumdodecylsulfat (SDS), 0,125 M Tris-HCl (pH 6,8) und 100 mM Dithiotreitol inkubiert. Die Proben wurden elektrophoretisch über 12,5 Massen% polyacrylamid-Trenngel 2,5 Stunden bei 65 mA/gel aufgetrennt (Laemmli, Nature 227: 680, 1971). Der eine Teil der Gelproben wurde mit Silbernitrat gefärbt, um die Polypeptide sichtbar zu machen. Der andere Teil der Proben wurde mittels der Immunoblottechnik untersucht, wobei Kaninchen-Antiserum verwendet wurde.

Die eluierten Fraktionen enthielten 42 mg Protein und 68 mg pre-S(2)-HBsAg. Damit wurde eine 145-fache Anreicherung des pre-S(2)-HBsAg erreicht.

Die nachfolgenden Beispiele 2 und 3 beschreiben die weitere Reinigung der in Beispiel 1 erhaltenen Fraktion.

Beispiel 2:

Eine nach Beispiel 1 erhaltene pre-S(2)-HBsAg-hältige Lösung wurde mittels Säulenchromatographie auf Sepharose 4B (Pharmacia) weiter gereinigt. Dazu wurde die Säule zunächst vorbereitet und mit 500 ml 0,02 M Tris (pH 7,0) bei einer Durchflußrate von 30 ml/Stunde gewaschen. Die pre-S(2)-HBsAg-hältige Lösung wurde gegen diesen Puffer dialysiert und das dialysierte Antigen, das in einer Konzentration von 10 mg/12 ml neben 6,2 mg Protein/12 ml vorlag, der Säule aufgetragen. Die Säule wurde mit 0,02 M Tris (pH 7,0) aktiviert und die eluierte pre-S(2)-HBsAg-hältige Fraktion enthielt 4,8 mg Protein und 8,2 mg pre-S(2)-HBsAg. "Silver-staining" und Immunoblotting der Polyacrylamidgele, die wie in Beispiel 1 beschrieben durchgeführt wurden, ergab eine Reinheit des pre-S(2)-HBsAg von mehr als 90 %.

Beispiel 3:

Eine nach Beispiel 1 erhaltene pre-S(2)-HBsAg-hältige Lösung wurde mittels Säulenchromatographie auf Lentil-Lectin-Sepharose 4B (Pharmacia) weiter gereinigt. Dazu wurde die säule zunächst vorbereitet und mit 100 ml 0,02 M Tris (pH 7,0) bei einer Durchflußrate von 50 ml/Stunde gewaschen. Die pre-S(2)-HBsAg-hältige Lösung wurde gegen diesen Puffer dialysiert und das dialysierte Antigen, das in einer Konzentration von 21 mg/25 ml neben 13 mg Protein/25 ml vorlag, der Säule mit einer Durchflußgeschwindigkeit von 25 ml/Stunde aufgetragen. Danach wurde das nicht adsorbierte Material mit Tris-Pufferlösung ausgewaschen und das pre-S(2)-HBsAg mit Tris-Pufferlösung, die 5 Massen% alpha-Methylmannosid enthielt, eluiert. Das Eluat enthielt 9,1 mg Protein und 15,6 mg pre-S(2)-HBsAg. "Silver-staining" und Immunoblotting der Polyacrylamidgele wurden, wie in Beispiel 1 beschrieben, durchgeführt und ergaben eine Reinheit des pre-S(2)-HBsAg von mehr als 90 %.

Die Reinigung von Hepatitis-B-Oberflächenantigenen, die statt des pre-S(2)-Teiles den pre-S(1)-Teil bzw. pre-S(2)- und pre-S(1)-Teile tragen, erfolgt analog den beschriebenen Ausführungsbeispielen. Das erfindungsgemäße Verfahren kann sogar zur Reinigung jedes Proteins herangezogen werden, das einen pre-S(1)- und/oder einen pre-S(2)-Teil des HBsAg trägt.

Die nachfolgenden Beispiele 4 bis 6 beschreiben die Verwendung verschiedener Trägermaterialien und -formen sowie Kopplungsmethoden die dem gewünschten Verwendungszweck entsprechen.

Beispiel 4:

Kopplung von monomerem Humanalbumin an Silikatträger

Als Träger kommen dabei entweder Kieselgele oder Mikro-Glaskugeln (controlled Pre Glass = CPG) in Frage.

10 g eines aminierten Glasträgers (Aminopropyl-CPG, Pierce) werden mit 100 ml einer 2,5 % wässerigen Glutaraldehydlösung vier Stunden bei Raumtemperatur geschüttelt. Nach der Aktivierung wird der Träger gründlich mit entionisiertem Wasser aldehydfrei gewaschen. Der so aktivierte Träger wird mit 200 mg humanem Serumalbumin in 0,1 M Phosphatpuffer (pH 8,0) eine Stunde lang inkubiert. Nach dem Blocken mit 1 M Äthanolamin und gründlichem Waschen mit Phosphatpuffer ist der Träger einsatzbereit. 200 ml eines pre-S(2)-HBsAg-hältigen Zellkuiturüberstandes (11 mg pre-S(2)-HBsAg, 950 mg Protein) werden mit 10 ml des Affinitätsträgers zwei Stunden lang bei 14°C inkubiert. Ungebundenes Protein wird durch Waschen mit Puffer auf einer Sinternutsche entfernt und das spezifisch gebundene pre-S(2)-HBsAg durch Inkubation des beladenen Trägers in 10 ml 8 M Harnstofflösung eluiert. Dieser Versuch ergab 6,8 mg hochreines pre-S(2)-HBsAg bei einer Gesamtproteinmenge von 5,4 mg (Bestimmung nach Bradford).

Beispiel 5:

Kopplung von monomerem Humanalbumin an Membranen

Als Träger kommen Membranen z.B. aus Nylon, Polyvinylidendifluorid oder Cellulose-Polyacrylamid-Mischpolymerisate in Frage. Nylon-Membran (z.B. Zetabind, CUNO) wird partiell durch Inkubation in $HCl/H_2O$ hydrolyisert. Die dabei freigesetzten Aminogruppen werden mit 1 M Oxaldialdehyd in Gegenwart von 0,1 M Natriumcyanoborhydrid bei pH7 zwei Stunden lang umgesetzt. Nach dem Waschen mit entionisiertem Wasser wird an diese mit reaktiven Aldehydgruppen aktivierte Membran humanes Serumalbumin (10 mg/ml in 0,1 M Phosphatpuffer pH7) in Gegenwart von 0,1 M Natriumcyanoborhydrid gekoppelt. Überschüssige reaktive Gruppen werden durch Zusatz von 1 M Äthanolamin geblockt. Nach dem Waschen mit Phosphatpuffer (0,1 M; pH8) kann die Membran zur selektiven Bindung von pre-S(2)-hältigen Proteinen verwendet werden.

Die Bindungskapazität einer so hergestellten Membran beträgt 5 bis 10 $\mu$g pre-S(2)-HBsAg/cm$^2$.

Beispiel 6:

Kopplung von monomerem Humanalbumin an Mikrotiterplatten

Es können dafür funktionalisierte Polystyrolplatten, wie z.B. Amino-Plate (mit primären Aminogruppen) oder Carbo-Plate (mit Carboxylgruppen) (Nissho Iwai Corp., Tokyo) oder ähnliche Materialien verwendet werden.

200 $\mu$l einer Lösung von N-Ethoxycarbonyl-2-ethoxy-1,2-dihydro-quinolin (40 mM in 50 % wässerigem Äthanol) werden in jedes Loch einer 96-Loch-Mikrotiterplatte pipettiert und zwei Stunden bei 40°C inkubiert. Nach sorgfältigem Waschen mit Äthanol und entionisiertem Wasser werden je 200 $\mu$l einer 1 % wässerigen Lösung von humanem Serumalbumin in jedes Loch pipettiert und über Nacht bei +4°C inkubiert. Nach dem Blocken mit 1 M Äthanolamin bzw. 1 M Acetatpuffer pH4 und darauffolgendem Waschen ist die Platte einsatzbereit.

100 $\mu$l eines pre-S(2)-HBsAg-hältigen Zellkulturüberstandes werden in steigenden Verdünnungen in die vorbereitete Mikrotiterplatte pipettiert und zwei Stunden bei +4°C inkubiert. Nach dem Waschen mit Puffer und Inkubation mit enzymmarkiertem Anti-pre-S(2)-HBsAg (monoklonale Antikörper) gemäß der bekannten ELISA-Technik wird durch Substratzugabe die gebundene Menge an pre-S(2)-Protein nach bekannten Methoden absorptionsspektroskopisch bestimmt.

Wird die Mikrotiterplatte mit SH-denaturiertem pre-S(2)-HBsAg oder synthetischem pre-S(2)-Peptid beladen, kann eine so behandelte Platte zur Bestimmung von Anti-pre-S(2)-Antikörpern (z.B. aus Patientenserum) verwendet werden.

10 mg pre-S(2)-HBsAg in 10 ml Phosphatpuffer (0,1 M; pH 7,0) werden mit 100 $\mu$l Mercaptoethanol in 10 % Natriumdodecylsulfat (SDS) 5 min bei 100°C inkubiert.

Dabei wird das immunologisch aktive S-Antigen des HBsAg zerstört (Milich D.R. et al., Enhauced immunogenicity of the Pre-S region of hepatitis B surface antigen, Science 227: 1195-1199 (1985)). Nach dem Blocken der SH-Gruppen mit 500 mg Jodacetamid für eine Stunde werden überschüssige Reaktions-

produkte durch Dialyse entfernt und das pre-S(2)-Antigen mit Phosphatpuffer pH 7,4 auf 100 $\mu$g Protein/ml verdünnt.

Eine vorbereitete HSA-Mikrotiterplatte wird mit dieser pre-S(2)-Lösung beschichtet und anschließend gewaschen. So präparierte Platten können auch in trockener Form gelagert werden.

Je 100 $\mu$l eines Anti-pre-S(2)-hältigen Kaninchenserums werden in steigenden Verdünnungen in die Löcher pipettiert. Nach Inkubation bei +4°C über Nacht werden gebundene pre-S(2)-Antikörper durch Inkubation mit enzymmarkiertem Anti-Kaninchenserum und nachfolgender Substratzugabe absorptionsspektroskopisch bestimmt.

Beispiel 7:

Bindungskapazität und Ausbeute an pre-S(2)-HBsAg bei Verwendung verschiedener Albumin-Träger-Komplexe Humanes Serumalbumin (HSA) bzw. polymerisiertes humanes Serumalbumin (poly-HSA) wurden in vergleichbarer Menge an verschiedene Trägermaterialien kovalent bzw. adsorptiv gebunden. Die Bindungskapazität und Ausbeute an pre-S(2)-HBsAg nach Elution wurden mittels einer HBsAg-spezifischen ELISA-Methode bestimmt und sind in der nachfolgenden Tabelle zusammengefaßt:

| Albumin | Träger | Art der Bindung | Kapazität mg/g Gel | % Ausbeute nach Elution mit | | | |
|---------|--------|-----------------|--------------------|--------|--------|--------|--------|
| | | | | 2M Hst | 4M Hst | 8M Hst | 3M KSCN |
| monomer | CNBr-Sepharose | kovalent über NH$_2$ | 7,6 | 45 | 85 | 60 | 50 |
| monomer | Anti-HSA Sepharose | adsorptiv | 0 | – | – | – | – |
| polymer | Anti-HSA Sepharose | adsorptiv | 5,8 | – | – | – | – |
| polymer | CNBr-Sepharose | kovalent über NH$_2$ | 6,4 | 12 | 40 | 55 | 45 |

Hst = Harnstoff

Der Tabelle ist zu entnehmen, daß sich mit dem erfindungsgemäßen unlöslichen polymeren Träger, bei dem im vorliegenden Ausführungsbeispiel das monomere Humanalbumin kovalent an Sepharose gebunden ist, höhere Ausbeuten an pre-S(2)-HBsAg erzielen lassen als mit Sepharose, an die Polyalbumin kovalent gebunden ist.

Diese deutlich höheren Ausbeuten sind auf die schonenderen Elutionsbedingungen zurückzuführen, die bei Verwendung des erfindungsgemäßen Trägers ermöglicht werden. Weiters ist ersichtlich, daß erst die kovalente Bindung des HSA an den Träger die erfindungsgemäße Eigenschaft der pre-S(2)-Affinität zur Folge hat und ein Molekularsieb, an die das Albumin lediglich adsorptiv gebunden ist, sich nicht zur affinitätschromatographischen Reinigung eignet.

**Patentansprüche**
**Patentansprüche für folgende Vertragsstaaten : AT, BE, CH, DE, DK, FR, GB, IT, LI, LU, NL, SE**

1. Komplex, bestehend aus einem unlöslichen polymeren Träger, insbesondere auf Agarose- oder Dextranbasis, an welchem Träger monomeres Humanalbumin kovalent gebunden ist, und einem pre-S-Hepatitis-B-Virus-Oberflächenantigen, welches über seinen pre-S(2)- und/oder pre-S(1)-Teil an den monomeren Humanalbumin in eluierbarer Form gebunden ist.

2. Unlöslicher polymerer Träger in einem Komplex nach Anspruch 1, an dem monomeres Humanalbumin kovalent gebunden ist.

3. Unlöslicher polymerer Träger nach Anspruch 2 auf Agarose- oder Dextranbasis.

4. Verfahren zur Reinigung von pre-S-Hepatitis-B-Virus-Oberflächenantigen unter Verwendung eines Komplexes nach Anspruch 1, dadurch gekennzeichnet,
   - daß der Komplex zur Entfernung von eventuell vorhandenen Verunreinigungen mit Pufferlösung gewaschen wird, und
   - daß das am monomeren Humanalbumin selektiv adsorbierte pre-S-Hepatitis-B-Virus-Oberflächenantigen abgespalten und gewonnen wird, indem der Komplex entweder mit einem Elutionsmittel behandelt wird, welches chaotrope Substanzen, wie Harnstoff, Guanidinhydrochlorid, Thiocyanat, Kaliumchlorid, Magnesiumchlorid oder Kaliumjodid, enthält; oder
   indem der Komplex mit Detergentien behandelt wird; oder
   indem der Komplex mit einem den pH-Wert verändernden Agens behandelt wird,
   wonach gegebenenfalls ein weiterer Reinigungsschritt vorgenommen wird.

5. Verfahren nach Anspruch 4, dadurch gekennzeichnet, daß als Detergentien ionische Detergentien, insbesondere Natrium-Deoxycholat und Taurodeoxycholsäure, oder zwitterionische Detergentien, insbesondere 3[(3-Cholamidopropyl)-dimethyl-ammonio]-1-propansulfonat und 3[(3-Cholamidopropyl)-dimethyl-ammonio]-2-hydroxy-1-propansulfonat,oder nicht-ionische Detergentien, insbesondere Octyl-Glucopyranoside, eingesetzt werden.

6. Verfahren nach Anspruch 4, dadurch gekennzeichnet, daß der weitere Reinigungsschritt in einer Gelfiltration, Ultrazentrifugation, hydrophoben Chromatographie, Lectin-Affinitätschromatographie oder in einer Ionenaustausch-Chromatographie besteht.

7. Verwendung eines Komplexes nach Anspruch 1 zur Herstellung von Diagnostika und Impfstoffen, wobei die Impfstoffe sowohl zur aktiven Immunisierung gegen Hepatitis B als auch zur Gewinnung von spezifischem Immunglobulin von Spendern, die mit solchen Impfstoffen immunisiert wurden, eingesetzt werden können.

**Patentansprüche für folgenden Vertragsstaat : ES**

1. Verfahren zur Herstellung eines Komplexes, bestehend aus einem unlöslichen polymeren Träger, monomerem humanmen Serumalbumin und einem pre-S-Hepatitis-B-Virus-Oberflächenantigen, dadurch gekennzeichnet, daß an den unlöslichen polymeren Träger monomeres Albumin kovalent gebunden wird und das pre-S-Hepatitis-B-Virus-Oberflächenantigen über seinen pre-S(2)- und/oder pre-S(1)-Teil an das monomere Humanalbumin in eluierbarer Form gebunden wird.

2. Verfahren nach Anspruch 1, dadurch gekennzeichnet, daß als unlöslicher polymerer Träger ein Träger auf Agarose- oder Dextranbasis zum Einsatz kommt.

3. Verfahren zur Herstellung eines Hepatitis-B-Virus-Diagnostikums oder -Impfstoffes, dadurch gekennzeichnet, daß ein preS-Hepatitis-B-Virus-Oberflächenantigen aus einem gemäß der Ansprüche 1 und 2 hergestellten Komplex eluiert ist und zu einer zur aktiven Immunisierung gegen Hepatitis wirksamen pharmazeutischen Präparation aufgearbeitet wird.

4. Verfahren zur Herstellung eines Hepatitis-B-Virus-Impfstoffes und -Diganostikums, dadurch gekennzeichnet, daß ein pre-S-Hepatitis-B-Virus-Oberflächenantigen aus einem gemäß der Ansprüche 1 und 2 hergestellten Komplex eluiert wird, zu einer zur aktiven Immunisierung gegen Hepatitis wirksamen pharmazeutischen Präparation aufgearbeitet wird, eine aktive Immunisierung herbeigeführt wird, die spezifischen Anti-Hepatitis-B-Virus-Immunglobuline gewonnen werden und zu einer zur passiven Immunisierung gegen Hepatitis-B-Virus wirksamen pharmazeutischen Präparation aufgearbeitet wird.

5. Verfahren zur Reinigung von pre-S-Hepatitis-B-Virus-Oberflächenantigen unter Verwendung eines Komplexes nach Anspruch 1, dadurch gekennzeichnet,
   - daß der Komplex zur Entfernung von eventuell vorhandenen Verunreinigungen mit Pufferlösung gewaschen wird, und
   - daß das am monomeren Humanalbumin selektiv adsorbierte pre-S-Hepatitis-B-Virus-Oberflächenantigen abgespalten und gewonnen wird, indem der Komplex entweder mit einem Elutionsmittel behandelt wird, welches chaotrope Substanzen, wie Harnstoff, Guanidinhydrochlorid, Thiocyanat, Kaliumchlorid, Magnesiumchlorid oder Kaliumjodid, enthält; oder indem der Komplex mit Detergentien behandelt wird; oder indem der Komplex mit einem den pH-Wert verändernden Agens behandelt wird,
   wonach gegebenenfalls ein weiterer Reinigungsschritt vorgenommen wird.

6. Verfahren nach Anspruch 5, dadurch gekennzeichnet, daß als Detergentien ionische Detergentien, insbesondere Natrium-Deoxycholat und Taurodeoxycholsäure, oder zwitterionische Detergentien, insbesondere 3[(3-Cholamidopropyl)-dimethyl-ammonio]-1-propansulfonat und 3[(3-Cholamidopropyl)-dimethyl-ammonio]-2-hydroxy-1-propansulfonat, oder nicht-ionische Detergentien, insbesondere Octyl-Glucopyranoside, eingesetzt werden.

7. Verfahren nach Anspruch 5, dadurch gekennzeichnet, daß der weitere Reinigungsschritt in einer Gelfiltration, Ultrazentrifugation, hydrophoben Chromatopgraphie, Lectin-Affinitätschromatographie oder in einer Ionenaustausch-Chromatographie besteht.

## Claims
## Claims for the following Contracting States : AT, BE, CH, DE, DK, FR, GB, IT, LI, LU, NL, SE

1. A complex comprised of an insoluble polymer carrier, in particular based on agarose or dextrane, having monomeric human albumin covalently bound thereto, and of a pre-S hepatitis B surface antigen bound in an elutable form to the monomeric human albumin by its pre-S(2) region and/or by its pre-S-(1) region.

2. An insoluble polymer carrier in a complex according to claim 1, to which monomeric human albumin is covalently bound.

3. An insoluble polymer carrier according to claim 2 based on agarose or dextrane.

4. A method of purifying pre-S hepatitis B surface antigen by using a complex according to claim 1, characterised in that
   - the complex is washed with buffer solution for removing possibly present impurities, and
   - the pre-S hepatitis B surface antigen selectively adsorbed to the monomeric human albumin is cleaved off and recovered either by treating the complex with an eluting agent containing chaotropic substances, such as urea, guanidine hydrochloride, thiocyanate, potassium chloride, magnesium chloride or potassium iodide; or
   by treating the complex with detergents; or
   by treating the complex with a pH modifying agent,
   whereupon, if desired, a further purification step is carried out.

5. A method according to claim 4, characterised in that, as the detergents, ionic detergents, in particular sodium desoxycholate and taurodesoxycholic acid, or zwitterionic detergents, in particular 3[(3-cholamidopropyl)-dimethyl-ammonio]-1-propane sulfonate and 3[(3-cholamidopropyl)-dimethyl-ammonio]-2-hydroxy-1-propane sulfonate, or non-ionic detergents, in particular octyl glucopyranosides, are used.

6. A method according to claim 4, characterised in that the further purification step consists in a gel filtration, ultracentrifugation, hydrophobic chromatography, lectin affinity chromatography or in an ion exchange chromatography.

7. The use of a complex according to claim 1 for preparing diagnostics and vaccines, wherein the vaccines may be used both for active immunization against hepatitis B and for obtaining specific immunoglobulin of donors immunized with such vaccines.

**Claims for the following Contracting State : ES**

1. A method of producing a complex comprised of an insoluble polymer carrier, monomeric human serum albumin and a pre-S hepatitis B surface antigen, characterised in that monomeric albumin is covalently bound to the insoluble polymer carrier, and the pre-S hepatitis B surface antigen is bound to the monomeric human albumin in elutable form by its pre-S(2) region and/or by its pre-S(1) region.

2. A method according to claim 1, characterised in that a carrier based on agarose or dextrane is used as the insoluble polymer carrier.

3. A method of producing a hepatitis B diagnostic or vaccine, characterised in that a pre-S hepatitis B surface antigen is eluted from a complex prepared according to claims 1 and 2 and is processed to a pharmaceutical preparation effective for active immunization against hepatitis.

4. A method of producing a hepatitis B vaccine and diagnostic, characterised in that a pre-S hepatitis B surface antigen is eluted from a complex prepared according to claims 1 and 2, is processed to a pharmaceutical preparation effective for active immunization against hepatitis, an active immunization is brought about, the specific anti-hepatitis B immunoglobulins are recovered and are processed to a pharmaceutical preparation effective for passive immunization against hepatitis B.

5. A method of purifying pre-S hepatitis B surface antigen by using a complex according to claim 1, characterised in that
   - the complex is washed with buffer solution for removing possibly present impurities, and
   - the pre-S hepatitis B surface antigen selectively adsorbed to the monomeric human albumin is cleaved off and recovered either by treating the complex with an eluting agent containing chaotropic substances, such as urea, guanidine hydrochloride, thiocyanate, potassium chloride, magnesium chloride or potassium iodide; or
   by treating the complex with detergents; or
   by treating the complex with a pH modifying agent,
   whereupon, if desired, a further purification step is carried out.

6. A method according to claim 5, characterised in that, as the detergents, ionic detergents, in particular sodium desoxycholate and taurodesoxycholic acid, or zwitterionic detergents, in particular 3[(3-cholamidopropyl)-dimethyl-ammonio]-1-propane sulfonate and 3[(3-cholamidopropyl)-dimethyl-ammonio]-2-hydroxy-1-propane sulfonate, or non-ionic detergents, in particular octyl glucopyranosides, are used.

7. A method according to claim 5, characterised in that the further purification step consists in a gel filtration, ultracentrifugation, hydrophobic chromatography, lectin affinity chromatography or in an ion exchange chromatography.

EP 0 419 446 B1

**Revendications**
**Revendications pour les Etats contractants suivants : AT, BE, CH, DE, DK, FR, GB, IT, LI, LU, NL, SE**

1. Complexe composé d'un support polymère insoluble, notamment à base d'agarose ou de dextrane, auquel de l'albumine humaine monomère est fixée par covalence, et d'un antigène de surface pré-S du virus de l'hépatite B, lequel est lié à l'albumine humaine monomère sous une forme éluable par l'intermédiaire de sa partie pré-S(2) et/ou pré-S(1).

2. Support polymère insoluble dans un complexe selon la revendication 1, auquel de l'albumine humaine monomère est fixée par covalence.

3. Support polymère insoluble selon la revendication 2, à base d'agarose ou de dextrane.

4. Procédé de purification de l'antigène de surface pré-S du virus de l'hépatite B par utilisation d'un complexe selon la revendication 1, caractérisé en ce que
   - on lave le complexe avec une solution tampon pour retirer les impuretés éventuellement présentes et
   - on sépare et on extrait l'antigène de surface pré-S du virus de l'hépatite B adsorbé sélectivement sur l'albumine humaine monomère,
      soit en traitant le complexe par un éluant qui contient des substances chaotropes telles qu'urée, chlorhydrate de guanidine, thiocyanate, chlorure de potassium, chlorure de magnésium ou iodure de potassium ;
      soit en traitant le complexe par des détergents;
      soit en traitant le complexe par un agent modifiant le pH,
      après quoi on procède le cas échéant à une étape de purification supplémentaire.

5. Procédé selon la revendication 4, caractérisé en ce qu'on utilise comme détergents des détergents ioniques, notamment du désoxycholate de sodium et de l'acide taurodésoxycholique, ou des détergents amphotères, notamment le 3-[(3-cholamidopropyl)diméthylammonio]-1-propane-sulfonate et le 3-[(3-cholamidopropyl)diméthylammonio]-2-hydroxy-1-propane-sulfonate, ou des détergents non ioniques, notamment des octylglucopyrannosides.

6. Procédé selon la revendication 4, caractérisé en ce que l'étape supplémentaire de purification consiste en filtration sur gel, ultracentrifugation, chromatographie hydrophobe, chromatographie d'affinité sur lectine ou chromatographie sur résine échangeuse d'ions.

7. Utilisation d'un complexe selon la revendication 1 pour la préparation de produits de diagnostic et de vaccins, les vaccins étant utilisables tant pour l'immunisation active contre l'hépatite B que pour l'obtention d'immunoglobuline spécifique de donneurs immunisés par de tels vaccins.

**Revendications pour l'Etat contractant suivant : ES**

1. Procédé de préparation d'un complexe composé d'un support polymère insoluble, de sérumalbumine humaine monomère et d'un antigène de surface pré-S du virus de l'hépatite B, caractérisé en ce que de l'albumine monomère est fixée par covalence au support polymère insoluble et que l'antigène de surface pré-S du virus de l'hépatite B est fixé à l'albumine humaine monomère sous une forme éluable par l'intermédiaire de sa partie pré-S(2) et/ou pré-S(1).

2. Procédé selon la revendication 1, caractérisé en ce qu'on utilise comme support polymère insoluble un support à base d'agarose ou de dextrane.

3. Procédé de préparation d'un produit de diagnostic ou d'un vaccin du virus de l'hépatite B, caractérisé en ce qu'on élue un antigène de surface pré-S du virus de l'hépatite B d'un complexe préparé selon les revendications 1 et 2 et qu'on le transforme en une préparation pharmaceutique efficace pour l'immunisation active contre l'hépatite.

4. Procédé de préparation d'un vaccin et d'un produit de diagnostic du virus de l'hépatite B, caractérisé en ce qu'on élue un antigène de surface pré-S du virus de l'hépatite B d'un complexe préparé selon

11

les revendications 1 et 2, qu'on le transforme en une préparation pharmaceutique efficace pour l'immunisation active contre l'hépatite, qu'on procède à une immunisation active, qu'on obtient les immunoglobulines spécifiques dirigées contre le virus de l'hépatite B et qu'on les transforme en une préparation pharmaceutique efficace pour l'immunisation passive contre le virus de l'hépatite B.

5. Procédé de purification de l'antigène de surface pré-S du virus de l'hépatite B par utilisation d'un complexe selon la revendication 1, caractérisé en ce que
   - on lave le complexe avec une solution tampon pour en retirer les impuretés éventuellement présentes et
   - on sépare et on extrait l'antigène de surface pré-S du virus de l'hépatite B adsorbé sélectivement sur l'albumine humaine monomère soit en traitant le complexe par un agent d'élution qui contient des substances chaotropes telles qu'urée, chlorhydrate de guanidine, thiocyanate, chlorure de potassium, chlorure de magnésium ou iodure de potassium ; soit en traitant le complexe par des détergents ; soit en traitant le complexe par un agent modifiant le pH,
     après quoi on procède le cas échéant à une étape de purification supplémentaire.

6. Procédé selon la revendication 5, caractérisé en ce qu'on utilise comme détergents des détergents ioniques, notamment le désoxycholate de sodium et l'acide taurodésoxycholique, ou des détergents amphotères, notamment le 3-[(3-cholamidopropyl)-diméthylammonio]-1-propane-sulfonate, ou le 3-[(3-cholamidopropyl)-diméthylammonio]-2-hydroxy-1-propane-sulfonate, ou des détergents non ioniques, notamment des octylglucopyrannosides.

7. Procédé selon la revendication 5, caractérisé en ce que l'étape supplémentaire de purification consiste en filtration sur gel, ultracentrifugation, chromatographie hydrophobe, chromatographie d'affinité sur lectine ou chromatographie sur résine échangeuse d'ions.